# EUROPEAN PATENT APPLICATION

(11) **EP 4 431 029 A1**
(43) Date of publication of application: **18.09.2024**
(21) Application number: 23162362.0
(22) Date of filing: 16.03.2023
(51) Int. Cl.: A61B 17/04

(54) **ANCHORS FOR TISSUE APPROXIMATION PROCEDURES**

(71) Applicant: Endo Tools Therapeutics S.A., 6041 Gosselies (BE)
(72) Inventor: The designation of the inventor has not yet been filed
(74) Representative: AWA Benelux

(57) **Abstract**

The present disclosure describes tissue anchors and assemblies for securing a suture thread in a tissue approximation procedure. The suture thread is provided with a plurality of protrusions (91) arranged along a length of the suture thread. The tissue anchor comprises an anchor body (11) having an elongate shape, a passageway (14) for the suture thread through the anchor body, and a pawl (15) adjacent to an outlet (141) of the passageway and configured to engage with the plurality of protrusions (91) so as to prevent movement of the suture thread in a first direction (92) from the outlet inwards into the anchor body. The pawl comprises a pawl body (151) pivotally coupled to the anchor body and the anchor body comprises a seat (111) for the pawl body, such that a force exerted by one of the plurality of protrusions on the pawl body in the first direction pivots the pawl body to abut on the seat.

## Description

### Technical field

The present invention is related to tissue anchors for securing suture threads for use in endoscopic tissue approximation procedures, particularly in the gastrointestinal tract, and to assemblies comprising such tissue anchors.

### Background art

Tissue anchors of the above kind are known from US 2004/0167546, US 2005/0251208 and WO 2007/140309. Known tissue anchors present several drawbacks. A first one is that they do not have optimal shape allowing easy deployment through a catheter. Secondly, they require dedicated instruments for performing suture tensioning. Thirdly, some anchors only allow securing the suture while an additional anchor is needed to press the tissue. Fourthly, some anchors may not provide desired securing force, or alternatively may not ensure complete tensioning of the suture thread as the force to pull the thread through the anchor increases by increasing tension on the suture thread, leading to incomplete tissue approximation. Particularly, at the beginning of a tissue approximation, the distal end of the suture is relatively loose, therefore pulling on the proximal end unlocks relatively easily the sliding insert and the tightening can begin. However, at the end of the tissue approximation, the suture is very much tensioned due to the compression of the tissues and therefore the suture tries to lock the sliding insert and may lock it before a proper tissue approximation is achieved.

### Summary

There is hence a need in the art to provide improved tissue anchors, allowing to overcome at least some of the above mentioned drawbacks. Particularly, there is a need to provide tissue anchors with a securing mechanism in which the force required to unlock the securing mechanism, e.g. to further tighten the thread, is not dependent on the tightening status. There is a need in the art to provide tissue anchors that provide improved mechanical strength and/or which are less prone to failure. There is a need in the art to provide tissue anchors which cause less trauma to tissue.

According to the present disclosure, there are therefore provided tissue anchors and assemblies comprising such anchors as set out in the appended claims. Solutions according to the present disclosure provide tissue anchors with a securing mechanism in which the force required to unlock the securing mechanism, e.g. to further tighten the thread, is not dependent on the tightening status.

According to a first aspect of the present disclosure, there is provided an assembly for securing a suture thread, such as in a tissue approximation procedure, particularly within a human or animal body, as set out in claim 1. A suture thread is provided with a plurality of protrusions arranged along a length of the suture thread, preferably spaced apart from one another along the length.

A tissue anchor comprises an anchor body having an elongate shape defining a longitudinal axis. The anchor body can be tubular along the longitudinal axis. A passageway for the suture thread through the anchor body comprises an outlet, such as through an external wall of the anchor body. The tissue anchor further comprises a pawl adjacent to the outlet. Preferably the pawl is arranged on an external wall of the anchor body. The pawl is configured to engage with the plurality of protrusions so as to prevent movement of the suture thread in a first direction from the outlet inwards into the anchor body and therefore provide for a thread locking mechanism. The pawl comprises a pawl body pivotally coupled to the anchor body. The anchor body comprises a seat for the pawl body, such that a force exerted by one of the plurality of protrusions on the pawl body in the first direction pivots the pawl body to abut on the seat.

An advantage of the above tissue anchor is that the seat provides improved back support for the pawl body to resist pressure by the suture thread. The pawl body will not bend inwards and halts when tissue reaction tries to push it backwards. This prevents the suture thread from being compressed more and more and eventually from being cut. A stronger and reliable thread securement can thereby be obtained, while the anchor can be slim and suitably long allowing to perform proper tissue anchoring and/or easy deployment through a catheter.

Advantageously, the hinge is arranged at a first end of the pawl body and the outlet is arranged at a second end of the pawl body opposite the first end, preferably wherein the first end and the second end define a direction which is substantially parallel to the longitudinal axis. This solution allows to have a sufficient lever to pivot the pawl body outwards during tensioning of the thread while not requiring much force to actuate. Less force is exerted on the hinge and if the hinge is formed integrally with the pawl body and the anchor body, excessive deformation of the hinge is prevented.

Advantageously, the seat comprises an abutting surface for the pawl, wherein the abutting surface is oblique to a plane parallel to a pivot axis of the pawl and parallel to the longitudinal axis. Advantageously, the abutting surface has mirror symmetry with respect to a plane perpendicular to the pivot axis. By providing such oblique and advantageously mirror symmetrical seats or abutment surfaces, an automatic alignment of the pawl body with respect to the anchor body is obtained in transverse direction (cross to longitudinal direction) and undesired sideways movement of the pawl body is prevented, which may deform the hinge, resulting in improved stability of the pawl body resting on the seat.

Advantageously, the pawl body comprises side edges at both sides of the pawl body between the first end and the second end. Advantageously, the side edges extend remote from the hinge to a position beyond the outlet, as considered along the longitudinal axis. The seat advantageously extends along both the side edges. Such a solution maximizes abutment surface area for the pawl body and improves mechanical strength of the thread locking mechanism and furthermore improves alignment capability by preventing sideways movement of the pawl body.

Advantageously, the hinge is arranged at a first end of the pawl body and the outlet is arranged at a second end of the pawl body opposite the first end along the longitudinal axis. The pawl body comprises an edge at the second end which is arcuate or curved, such as in projection on a plane parallel to the longitudinal axis. Such a solution further improves self-aligning capability by preventing sideways movement of the pawl body.

Advantageously, the pawl body comprises a narrower section at a first end of the pawl body and a larger section at a second end opposite the first end along the longitudinal axis. The pawl body is pivotally coupled to the anchor body at the first end. A hinge can be formed integrally with the pawl body and the anchor body, i.e. made from a single part or workpiece, such that the pawl body resembles a flap. Such a narrower section allows on the one hand to have a hinge that can be actuated with less force, while the larger section provides enlarged contact area with the seat. When the pawl body is furthermore arcuate shaped, mechanical resistance is even further improved.

Advantageously, the tissue anchor is a T-type anchor with the outlet for the suture thread located in a central section of the anchor body intermediate opposite first and second end sections of the anchor body along the longitudinal axis. The central section comprises a point of weakness configured to act as a pivot for the first and second end sections of the anchor body, allowing the first and second end sections to flex relative to the central section. Advantageously, a cross-sectional area of the anchor body in the point of weakness is reduced compared to the first and second end sections. This allows the end sections of the anchor body to flex on the point of weakness, such as about an axis substantially cross to the longitudinal axis. As a result, the tissue anchor complies better to the tissue and trauma to the tissue can be reduced or avoided. In some examples, the reduced thickness is obtained by a cut out or recess in the anchor body adjacent the inlet, e.g. about an edge of the inlet.

According to a second aspect of the present disclosure, there is provided an assembly for securing a suture thread in a tissue approximation procedure for a human or animal body, wherein the assembly comprises a suture thread, a first tissue anchor and a second tissue anchor, advantageously the first and the second tissue anchors being configured to approximate tissue between the first and the second tissue anchors. The second tissue anchor is elongate defining a second longitudinal axis, wherein the suture thread is secured to the second tissue anchor in a central section of the second tissue anchor intermediate opposite first and second end sections of the second tissue anchor along the second longitudinal axis. Advantageously, the central section comprises a point of weakness allowing the first and second end sections of the second tissue anchor to flex relative to the central section of the second tissue anchor. Advantageously, the second tissue anchor comprises a transverse cut in the central section that forms the point of weakness. Advantageously, the first tissue anchor comprises a thread locking mechanism allowing the first tissue anchor to be advanced along the suture thread towards the second tissue anchor while preventing movement of the first tissue anchor away from the second tissue anchor along the tissue. Advantageously, the first tissue anchor is as according to the first aspect.

According to a further aspect of the present disclosure, an assembly for securing a suture thread in a tissue approximation procedure is provided. Such an assembly comprises a suture thread and at least one tissue anchor configured to secure the suture thread, wherein the suture thread comprises a frangible portion. Advantageously, the at least one tissue anchor comprises an anchor body and a passageway for the suture thread through the anchor body. The tissue anchor comprises an unidirectional securing means or system for the suture thread configured to allow movement of the suture thread relative to the tissue anchor in one direction while preventing movement of the suture thread relative to the tissue anchor in a second direction opposite the first direction.

One advantage of the assemblies of the above kind is that no additional instrument is required to cut the thread at the end of a tissue approximation procedure. Furthermore, it prevents excessive force to be exerted on the tissue anchor which may deform the anchor and cause malfunction.

A method for approximating tissue in a human or animal body is described herein. The method comprises a first operation of endoscopically forming a tissue fold in the body, particularly in a cavity of the gastrointestinal tract, more particularly in the stomach. In a second operation, the tissue fold is pierced with a deployment device defining a lumen. An assembly according to the present disclosure is accommodated in the lumen. In a third operation, a first one of at least the first and second tissue anchors is deployed through the lumen across the tissue fold. In a fourth operation, at least one further tissue fold is endoscopically formed and the at least one further tissue fold is pierced with the deployment device. In a fifth operation, a second one of at least the first and second tissue anchors is deployed through the lumen across the further tissue fold. In a sixth operation, the first and at least one further tissue folds are approximated by approximating the first and second tissue anchors towards one another, thereby securing the tissue folds between the first and second tissue anchors. In an optional seventh operation, the suture thread is cut, such as at a frangible portion.

### Brief description of the drawings

Aspects of the invention will now be described in more detail with reference to the appended drawings, wherein same reference numerals illustrate same features and wherein:
Figure 1 represents a perspective view of an embodiment of a tissue anchor according to the present disclosure;
Figure 2 represents a plan view and a cross section view along section line A-A of the tissue anchor of Fig. 1;
Figure 3 represents a perspective view of another embodiment of a tissue anchor according to the present disclosure;
Figure 4 represents a plan view of the tissue anchor of Fig. 3;
Figure 5 represents a tissue anchor assembly according to aspects of the present disclosure;
Figure 6 represents the tissue anchor of Fig. 1 when deformed to comply with approximated tissue;
Figure 7 represents a perspective view of a fixed tissue anchor according to the present disclosure;
Figure 8 represents a plan view of the fixed tissue anchor of Fig. 7;
Figure 9 represents the fixed tissue anchor of Fig. 7 when deformed to comply with approximated tissue;
Figure 10 represents a diagram of a tissue fold that is pierced by a deployment device including a lumen for deploying the tissue anchors and assemblies of the present disclosure;
Figure 11 represents a diagram of multiple tissue folds being approximated by assemblies of the present disclosure.

### Detailed description

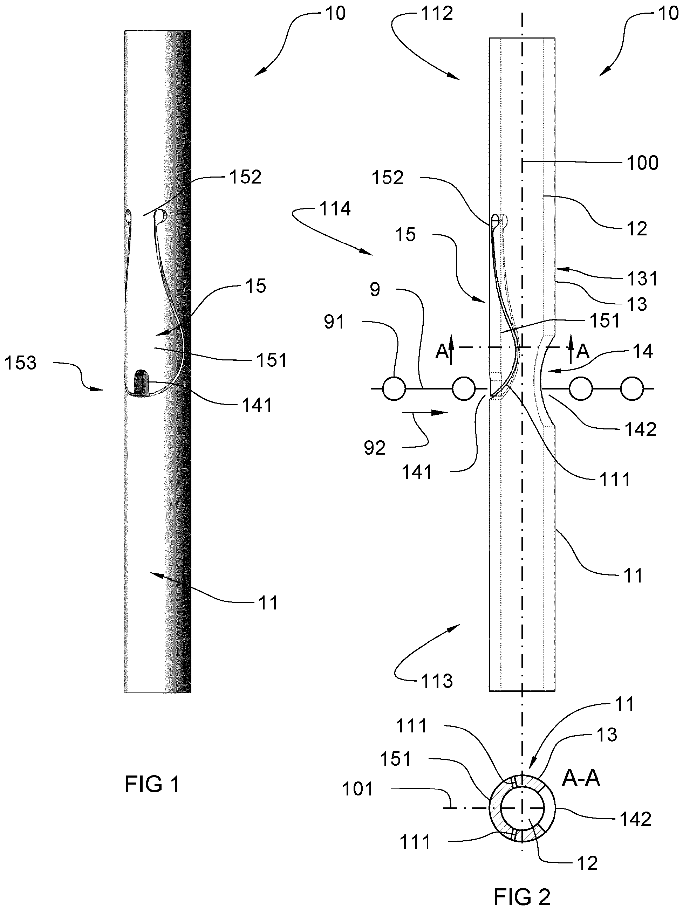

Referring to Figs. 1 and 2, a tissue anchor 10 comprises an anchor body 11 having an elongate shape defining a longitudinal axis 100. The anchor body 11 is preferably tubular with internal channel 12 and wall 13 defining the channel 12 and additionally define the external wall 131 of the anchor body. A passageway 14 for the suture thread 9 through the anchor body comprises an inlet 142 and an outlet 141 through the wall 13. Inlet 142 and outlet 141 define a preferably straight suture thread passageway 14 oriented transverse (perpendicular) to longitudinal axis 100. The tissue anchor 10 further comprises a pawl 15 adjacent to the outlet 141 arranged on the wall 13 of the anchor body. The pawl 15 is configured to engage with the protrusions 91 of the suture thread 9 so as to prevent movement of the suture thread in a first direction 92 from the outlet 141 inwards into the anchor body while allowing movement of the suture thread in the opposite direction.

The pawl 15 comprises a pawl body 151 pivotally coupled to the anchor body 11 via a hinge 152 which can be formed integrally with the anchor body 11 and the pawl body 151. The anchor body comprises a seat 111 for the pawl body 151, e.g., comprising or consisting of an abutting surface, such that a force exerted by one of the plurality of protrusions 91 on the pawl body in the first direction 92 pivots the pawl body 151 to abut on the seat 111. The seat 111 is advantageously located between the hinge 152 and the outlet 141.

The pawl body 151 is preferably cut out of the anchor body 11, e.g. through laser cutting or wire electric discharge machining. In case anchor body 11 is tubular, pawl body 151 is formed by a portion of the wall 13 cut out from anchor body. Pawl body 151 is advantageously still attached to the anchor body (wall 13) at hinge portion 152 and advantageously forms a flap connected to the anchor body only via the hinge portion 152. Hinge portion 152 can be formed by one or more strips connecting the pawl body 151 to the anchor body 11. Advantageously, hinge 152 is formed as an integral piece with anchor body 11 and pawl body 151, e.g. hinge 152 is a piece of the (tubular) anchor body 11 that is not cut out.

Anchor body 11 is advantageously tubular (cylindrical or prismatic) with internal channel 12 extending along the longitudinal axis 100. Pawl body 151 advantageously has a teardrop-like shape, with pawl body 151 being wider and the section adjacent to the hinge 152 being narrower (as seen in cross sections perpendicular to the longitudinal axis) . The narrower section of hinge 152 allows for easy pivoting of the pawl body 151, such as on a tubular or cylindrical anchor. The cross section of pawl body 151 transverse to axis 100 is advantageously curved or arcuate, which increases mechanical strength when the protrusions 91 press on the pawl body 151 in the direction 92.

Advantageously, the seat 111 comprises an abutting surface configured to accommodate or engage with the pawl body 151 which abutting surface at least partly lies in a plane that is oblique to a plane parallel to longitudinal axis 100 and to the pivot axis of the hinge 152, providing increased strength. As seen more clearly in the section A-A of Fig. 2, the seat 111 advantageously has mirror symmetry with respect to a median plane 101 of the pawl body 151 perpendicular to the pivot axis of the hinge 152 and advantageously parallel to the longitudinal axis 100 to align the pawl body 151 in the seat 111. This can prevent sideways movement of the pawl and aids to self-align the pawl body with respect to the seat. Additionally, the surface of seat 111 is advantageously oriented in a direction departing from a radial direction of the anchor body to increase seating surface and/or improving self-aligning capability of the pawl, i.e. the surface of seat 111 is oblique with respect to the local radial direction of the anchor body or is oriented in a direction not parallel with the local radial direction. By way of example, the seating surface can be oblique to a local normal direction on the external surface of the anchor body.

The pawl body 151 can comprise further self-aligning capabilities. In some examples, the pawl body 151 has mirror symmetry with respect to a median plane parallel to (and possibly including) the longitudinal axis 100 and advantageously cross to (perpendicular to) a pivot axis of hinge 152. In addition, or alternatively, the free end 153 of the pawl body opposite the hinge section 152 can have a curved or arcuate edge, e.g. as seen from the direction 92, and the seat 111 can have a complementary shape to support the curved or arcuate edge of the free end 153 of the pawl body 151.

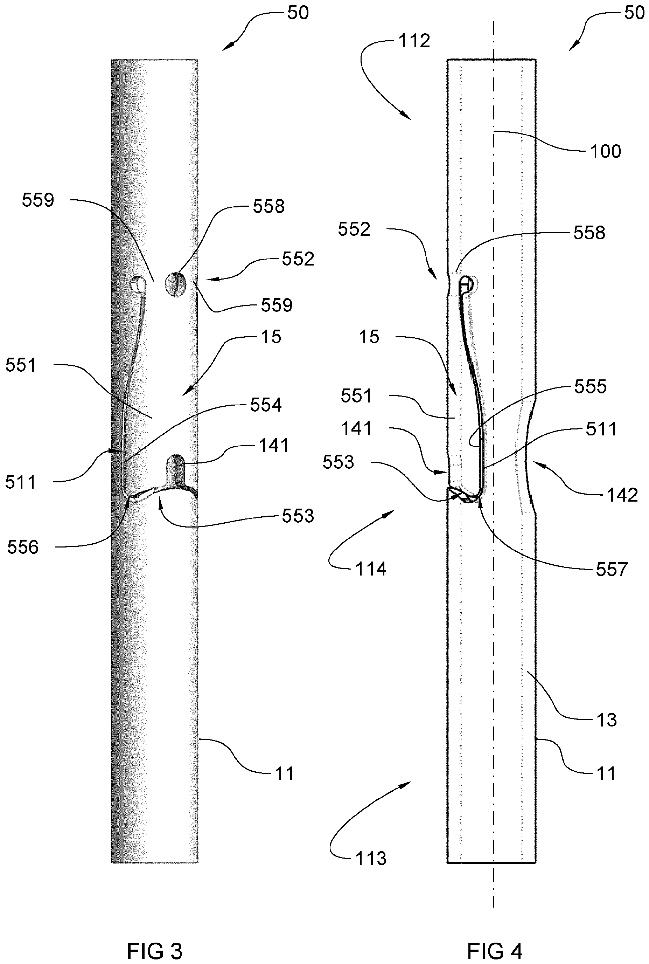

Referring to Figs. 3 and 4, another example of tissue anchor 50 according to the present disclosure differs from the tissue anchor 10 described above in the shape of the pawl 15. Pawl 15 comprises a pawl body 551 with a more elongate shape compared to pawl body 151. Particularly, pawl body 551 extends from the hinge section 552 to a free end 553 that extends beyond the outlet 141 when considered along the longitudinal axis. This allows for an extended seat 511 and hence increased abutting surface for the pawl body 551 compared to pawl body 151. Specifically, pawl body 551 comprises left and right side edges 554, 555 respectively which extend, advantageously from the hinge section 552, to respective extremities 556, 557 at the free end 553. The extremities 556, 557 of the side edges are located remote from the hinge section and beyond the position of the outlet 141, along the longitudinal axis 100. The free end 553 advantageously deploys from the extremities 556, 557 at the side edges back towards the hinge section in the centre, where the free end 553 advantageously borders the outlet 141, and it advantageously does so along a curved or arcuate edge. By so doing, a length of an abutting surface on the seat 511 can be maximized for improved mechanical strength. Additionally, such a shape of the free end 553 improves self-aligning capability of the pawl body 551 with respect to the anchor body. In some examples, the free end 553 and a corresponding edge of the seat (anchor body) comprise abutting surfaces that are oblique (i.e., not perpendicular) to the longitudinal axis 100 to even further improve self-aligning capabilities. Pawl body 551 can otherwise have a same configuration as pawl body 151 as described in relation to Figs. 1-2, e.g. with an arcuate or curved cross section in a plane perpendicular to the longitudinal axis 100 and/or with side edges 554, 555 and corresponding abutting surface of seat 511 being oblique to a local radial direction of the anchor body 11. It will be appreciated that pawl body 551, like pawl body 151 advantageously has mirror symmetry with respect to a median plane parallel to (and possibly including) the longitudinal axis 100 and advantageously cross to (perpendicular to) a pivot axis of hinge 552.

It will be appreciated that the seat can have serrations or teeth to allow increasing the contact area between pawl and anchor body. In that case, the pawl body 151 will have an edge provided with serrations or teeth to complement the shape of the seat.

It is possible to increase the width of the hinge section 152 while providing additional cuts through the hinge section to provide appropriate weakening to reduce the force for pivoting the pawl body, particularly when the wall of the anchor body is curved or arcuate in a plane perpendicular to the longitudinal axis. It will be appreciated that the hinge section 552 of tissue anchor 50 is somewhat wider compared to the hinge section 152 of pawl body 151 in Figs. 1-2. Hinge section 552 comprises a through-cut or through-opening 558 through the wall 13 of the anchor body 11 to reduce the effort for pivoting the pawl body 551 about the hinge, and additional such (through-)cuts may be provided as desired. Due to the through-cut 558, the pawl body 551 is connected to the anchor body 11 via two slender strip members 559 which are made as an integral piece with pawl body 551 and anchor body 11 such as by appropriate cutting operation through the anchor body.

The outlet 141 can be provided as a cavity or aperture at an edge of the pawl body 151 or 551, or at an edge of the anchor body 11 (seat 111), or a combination of both. Advantageously, the outlet 141 is positioned between the pawl body 151, 551 and a wall 13 of the anchor body. Specifically, the outlet 141 is surrounded or bordered in part by the pawl body 151, 551, such as at the free end 153, 553 and in part by an inner edge of the anchor body 11 (of the wall 13).

Tissue anchor 10, 50 is hence advantageously a T-type anchor. Specifically, the inlet and outlet passageways 142, 141 respectively, of the suture thread are advantageously aligned along the transverse direction.. The passageway 14 is advantageously located in a central section 114 of the tissue anchor (anchor body 11) intermediate the end sections 112, 113 of the tissue anchor located at opposite extremities of the tissue anchor (anchor body) along the longitudinal axis 100. Advantageously, the inlet 142 and outlet 141 of passageway 14 are located at about mid-length of a length of the tissue anchor.

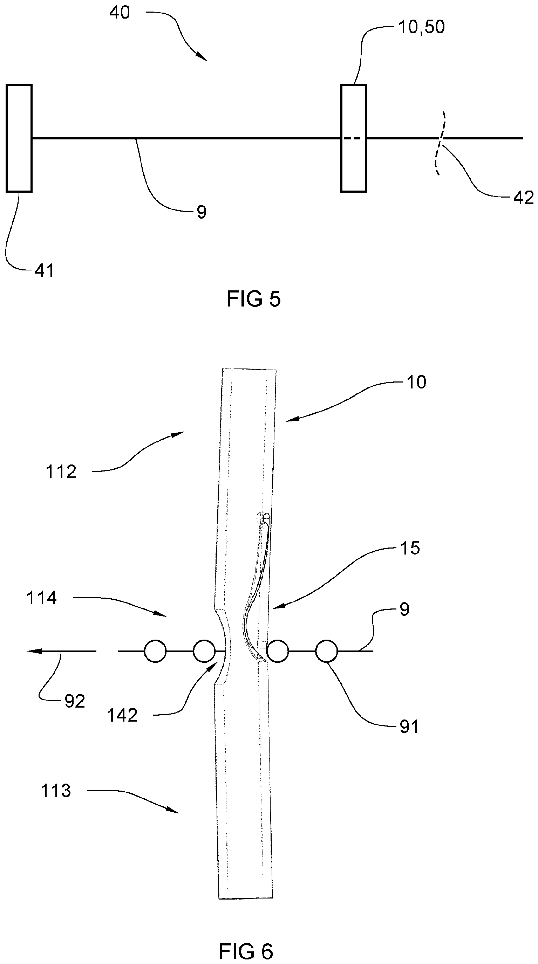

The anchor body 11 advantageously has a reduced cross-sectional area (in a plane perpendicular to the longitudinal axis 100) in a region of the passageway 14, particularly, in the region of the outlet 141 (region of the pawl 15) and/or of the inlet 142. Particularly, the inlet 142 can comprise a recess or inlet bore that is appropriately larger than the size of the protrusions 91 of the suture thread 9 to (further) reduce cross-sectional area of the anchor body and/or the anchor body 11 can comprise a transverse cut at a position corresponding to the inlet and/or outlet of the passageway (not shown). This advantageously creates a point of weakness of the anchor body at a position corresponding to the passageway 14, providing for a pivot in the central section 114 that allows the end sections 112, 113 of the tissue anchor to flex relative to the central section 114 about an axis transverse to the longitudinal axis, as illustrated in Fig. 6. When the tissue anchor is tightened against the tissue (located at the side of the inlet 142 of passageway 14), the force exerted by protrusion 91 on the region around the outlet 141 in the central section 114, causes a deformation of the tissue anchor in which the end sections 112, 114 flex away from the tissue relative to the central section 114. This allows for reducing or avoiding trauma to the tissue due to a too rigid anchor. The flexibility of the anchor body is even improved when the passageway 14 is substantially straight and transverse to the longitudinal axis 100, and particularly when the pawl body 151 is formed as a flap cut out from the anchor body which reduces the cross-section of the anchor body in the region of the outlet 141. The flexibility can be improved even further by manufacturing the anchor body from a flexible material, such as Nitinol (nickel titanium alloy).

Tissue anchors of the present disclosure can be manufactured from any appropriate material, in particular metal, such as nickel titanium alloys, or medical grade plastic materials.

Tissue anchors of the present disclosure can be easily deployed through a catheter procedure as they allow to be easily loaded in small instrument channels of catheter devices. Referring to Fig. 5, in typical anchor assemblies 40, at least two tissue anchors are utilized, and a first tissue anchor 10, 50 can be pushed along suture thread 9 towards a second tissue anchor 41 by any suitable device, such as a push rod, to approximate tissue between the first and second anchors. The first tissue anchor can be utilized to tighten the suture thread by the unidirectional locking mechanisms as described in the present disclosure. Advantageous applications are tissue approximations in the gastrointestinal tract, specifically gastric reduction procedures.

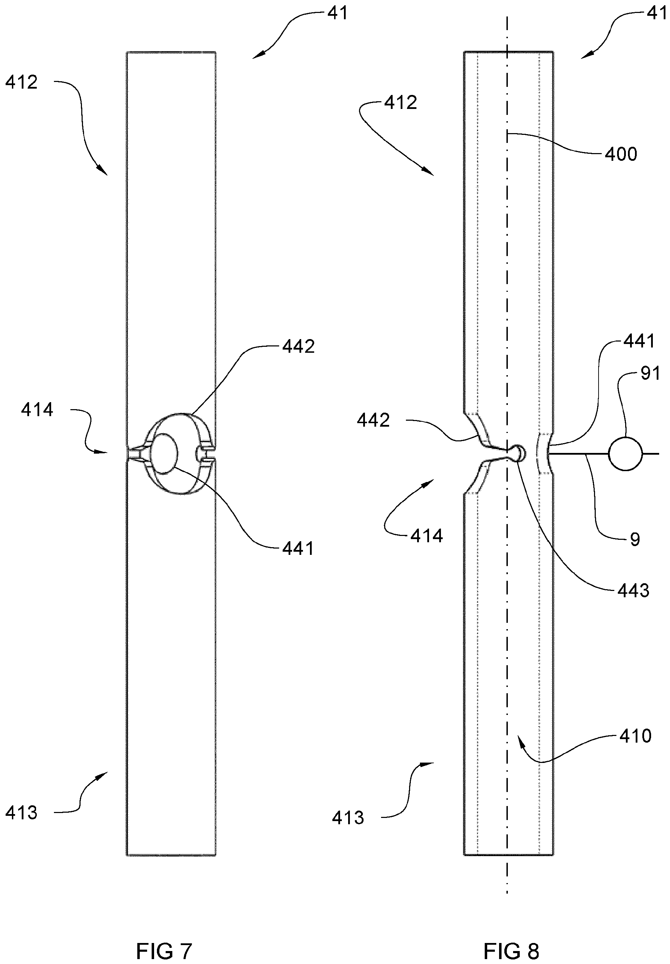

Referring to Figs. 7-8, the second tissue anchor 41 is advantageously a T-type anchor being elongate along longitudinal axis 400. Tissue anchor 41 can be tubular with cylindrical or prismatic base and lumen 410. The suture thread 9 is possibly fixedly secured to the tissue anchor 41, such as in a central section 414 intermediate end sections 412, 413 of the tissue anchor located at longitudinal extremities thereof. In some examples, the tissue anchor 41 comprises an outlet hole 441 for the suture thread 9, such as in central section 414, and possibly an inlet hole 442 opposite the outlet hole 441. The outlet hole 441 is advantageously smaller than a protrusion 91 securing the suture thread to the tissue anchor 41. The inlet hole 442 can be larger than the protrusion 91 to facilitate assembly. The inlet hole 442 can alternatively be dispensed with and the thread inserted via the lumen 410.

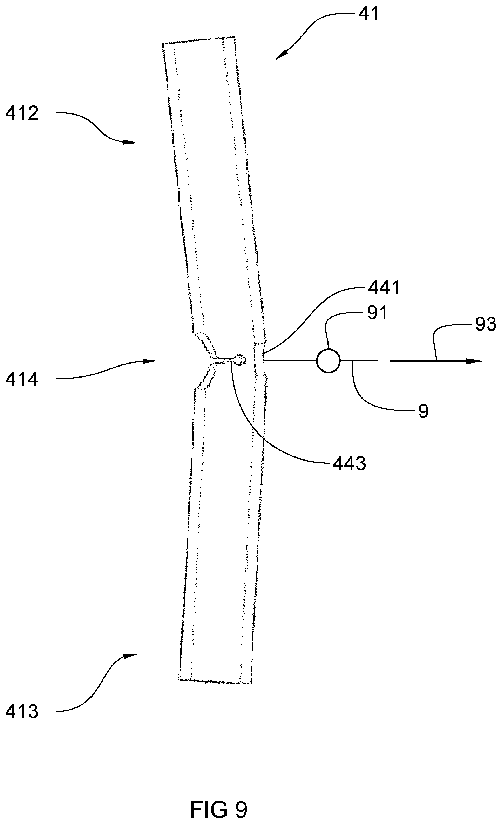

Like the tissue anchors 10, 50, the second tissue anchor 41 can comprise a point of weakness in the central section 414, particularly at a position corresponding to the inlet hole 442 and/or outlet hole 441, that allows the end sections 412, 413 to flex and comply with the tissue that is tightened or approximated. In some examples, the point of weakness comprises a cut 443 transverse through the body of the tissue anchor and particularly at a longitudinal position corresponding to the inlet and/or outlet hole. This reduces a cross-sectional area of the anchor body in the central section to create a pivot as illustrated in Fig. 9, with pivot axis transverse to the longitudinal axis 400. When a force is exerted on the suture thread 9 in direction of the arrow 93 due to approximated tissue, the point of weakness 443 allows the end sections 412, 413 to flex in opposite direction compared to the force direction 93, providing better compliance to tissue and reducing or avoiding trauma.

The suture thread 9 can comprise a frangible portion 42 configured to break when stress or tension in the thread 9 exceeds a threshold. Such a frangible portion can be obtained by several means, such as one or a combination of:
- heat treating and/or mechanically deforming a section of the thread;
- abutting two lengths of suture with a weak junction;
- in the case of braided suture, cutting part of the braiding at one spot;
- in the case of monofilament suture, draw the suture to have a locally smaller diameter;
- laser cutting to remove part of the suture or adding stress risers.

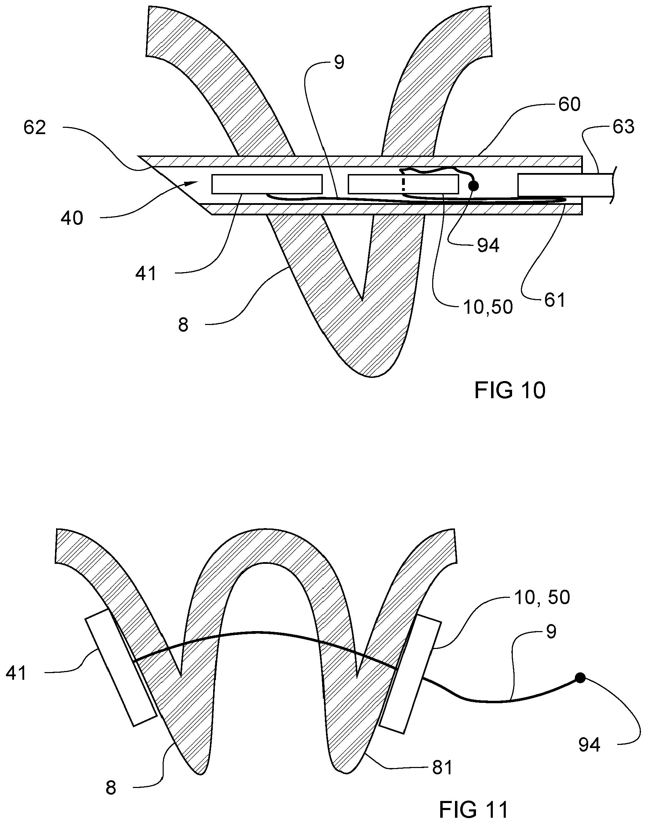

Referring to Figs. 10-11, to approximate tissue utilizing assemblies according to the present disclosure, a tissue fold 8 is formed endoscopically, particularly in a cavity of a human or animal body, such as in a cavity of the gastrointestinal tract, e.g. the stomach. Tissue folds may be formed by utilizing known instruments such as a grasper. A deployment device 60, such as including a piercing needle 62 and a lumen 61 is advanced through the human or animal body, such as through an endoscope or catheter (not shown). The tissue fold 8 is pierced with the needle 62 and the deployment device 60 advanced across the tissue fold. A tissue anchor, such as the second tissue anchor 41 is deployed from the lumen 61 across the tissue fold 8, e.g. utilizing a push device 63, such as a push rod. Next, the deployment device 60 is retracted from the tissue fold 8. At this stage, advantageously, another tissue anchor, such as the first tissue anchor 10, 50 is still housed in the lumen 61.

A further tissue fold 81 can be formed endoscopically and deployment device 60 made to pierce and advance across the further tissue fold 81. Another tissue anchor, such as the first tissue anchor 10, 50 is deployed from the lumen 61 across the further tissue fold 81. Next, the deployment device 60 is retracted from the further tissue fold, to obtain the situation illustrated in Fig. 11, in which a plurality of tissue folds 8, 81 (may be more than two) are interposed between the first and second tissue anchors. At this stage, the first tissue anchor 10, 50 can be approximated towards the second tissue anchor 41 by advancing the first tissue anchor along the suture thread 9. In some examples, a grasping device engages the thread 9, e.g. at protrusion 94, a loop or lasso to stretch the thread 9 while a push device, such as a push rod, approximates the first tissue anchor 10, 50 towards the second tissue anchor 41. The first tissue anchor is secured on the suture thread by the thread locking mechanisms as described above (not shown in Figs. 10-11 for clarity). Once the tissue anchors are in place, the suture thread can be cut, such as at a frangible portion.

It will be appreciated that the assemblies according to the present disclosure can comprise more than two tissue anchors, particularly attached to a same suture thread. At least one of these tissue anchors can be as any one of the first tissue anchor described herein and at least one of them can be as the second tissue anchor as described herein.

## Claims

1. Assembly for securing a suture thread in a tissue approximation procedure, the assembly comprising a suture thread (9) and a first tissue anchor (10, 50),
wherein the suture thread is provided with a plurality of protrusions (91) arranged along a length of the suture thread,
wherein the first tissue anchor comprises:
an anchor body (11) having an elongate shape defining a longitudinal axis (100),
a passageway (14) for the suture thread through the anchor body, the passageway comprising an outlet (141), and
a pawl (15) adjacent to the outlet and configured to engage with the plurality of protrusions (91) so as to prevent movement of the suture thread in a first direction (92) from the outlet inwards into the anchor body,
wherein the pawl comprises a pawl body (151, 551) pivotally coupled to the anchor body,
wherein the anchor body comprises a seat (111, 511) for the pawl body, such that a force exerted by one of the plurality of protrusions on the pawl body in the first direction pivots the pawl body to abut on the seat.

2. Assembly of claim 1, wherein the pawl comprises a hinge (152) coupled to the anchor body, wherein the hinge (152) is arranged at a first end of the pawl body (151) and the outlet (141) is arranged at a second end (153, 553) of the pawl body opposite the first end, wherein the seat (111) is arranged between the hinge (152) and the outlet (141), wherein the first end and the second end define a direction which is substantially parallel to the longitudinal axis (100).

3. Assembly of claim 2, wherein the pawl body (151, 551) comprises side edges (554, 555) at both sides of the pawl body between the first end and the second end (153, 553), wherein the side edges (554, 555) extend remote from the hinge (152) to a position beyond the outlet (141), and wherein the seat (111, 553) extends along the side edges.

4. Assembly of any one of the preceding claims, wherein the hinge (152) is arranged at a first end of the pawl body (151, 551) and the outlet (141) is arranged at a second end (153 553) of the pawl body opposite the first end along the longitudinal axis, wherein the pawl body comprises an edge at the second end which is arcuate or curved in projection on a plane parallel to the longitudinal axis (100), preferably the plane is additionally parallel to a pivot axis of a hinge (152, 552) between the pawl body and the anchor body.

5. Assembly of any one of the preceding claims, wherein the anchor body (11) comprises an external wall (13) circumferential about the longitudinal axis (100), wherein the pawl body (151, 551) defines a portion of the external wall, and wherein a cross-section of the pawl body in a plane perpendicular to the longitudinal axis has substantially a curved or arcuate shape, preferably the external wall has normal vectors perpendicular to the longitudinal axis, preferably the pawl body and the anchor body define the external wall in a complementary fashion, preferably wherein the external wall is essentially cylindrical or prismatic with a base arranged in a plane perpendicular to the longitudinal axis.

6. Assembly of any one of the preceding claims, wherein the pawl body (151, 551) defines a first portion of a circumferential edge of the outlet (141) and wherein the anchor body (11) defines a second portion of the circumferential edge.

7. Assembly of any one of the preceding claims, wherein the pawl body (151) is formed as a flap partially cut out of the anchor body, preferably wherein the anchor body (11) is at least partially tubular having a lumen extending along the longitudinal axis and wherein the pawl body allows access to the lumen.

8. Assembly of claim 7, wherein the pawl body (151) is pivotally connected to the anchor body by a strip, wherein the strip comprises one or more through-cuts for weakening the strip to facilitate pivotal movement.

9. Assembly of any one of the preceding claims, wherein the pawl body (15) comprises a narrower section at a first end of the pawl body and a larger or wider section at a second end opposite the first end along the longitudinal axis, wherein the pawl body is pivotally coupled to the anchor body at the first end, preferably the pawl body is formed integrally with the anchor body, preferably wherein the narrower section is configured to form a weakened portion so as to operate as a hinge for the pawl body.

10. Assembly of any one of the preceding claims, wherein the passageway (14) extends substantially cross to the longitudinal axis (100) and is located in a central section (114) of the anchor body intermediate opposite first and second end sections (112, 113) of the anchor body along the longitudinal axis.

11. Assembly of any one of the preceding claims, wherein the outlet (141) is located in a central section (114) of the anchor body intermediate opposite first and second end sections (112, 113) of the anchor body along the longitudinal axis, wherein the central section (114) comprises a point of weakness configured to act as a pivot for the first and second end sections (112, 113) of the anchor body, allowing the first and second end sections to flex relative to the central section, preferably wherein a cross-sectional area of the anchor body in the point of weakness is reduced compared to the first and second end sections (112, 113).

12. Assembly of any one of the preceding claims, wherein the plurality of protrusions (91) are knots or beads attached to the suture thread (9).

13. Assembly (40) of any one of the preceding claims, further comprising a second tissue anchor (41) attached to the suture thread, wherein the first and the second tissue anchors being configured to approximate tissue between the first and the second tissue anchors.

14. Assembly of the preceding claim, wherein the second tissue anchor (41) is elongate defining a second longitudinal axis (400), wherein the suture thread (9) is secured to the second tissue anchor in a central section (414) of the second tissue anchor intermediate opposite first and second end sections (412, 413) of the second tissue anchor along the second longitudinal axis, wherein the central section comprises a point of weakness (443) allowing the first and second end sections of the second tissue anchor to flex relative to the central section (414) of the second tissue anchor, preferably wherein the second tissue anchor comprises a transverse cut in the central section that forms the point of weakness.

15. Assembly (40) of any one of the preceding claims, wherein the suture thread comprises a frangible portion (42), wherein the frangible portion (42) is a point of weakness configured to break when a stress in the suture thread exceeds a predetermined threshold.
